# EUROPEAN PATENT SPECIFICATION

(11) **EP 2 560 475 B1**
(45) Date of publication and mention of the grant of the patent: **14.02.2018**
(21) Application number: 11772785.9
(22) Date of filing: 22.04.2011
(51) Int. Cl.: A01H 5/00, A01H 5/10, C12N 5/04, A01N 63/02, C12N 15/82

(54) **COMBINATIONS INCLUDING CRY34AB/35AB AND CRY3AA PROTEINS TO PREVENT DEVELOPMENT OF RESISTANCE IN CORN ROOTWORMS (DIABROTICA SPP.)**
KOMBINATIONEN MIT CRY34AB/35AB- UND CRY3AA-PROTEINEN ZUR VERHINDERUNG DER ENTWICKLUNG EINES WIDERSTANDS IN MAISWURZELBOHRERN (DIABROTICA SPP.)
COMBINAISONS INCLUANT LES PROTÉINES CRY34AB/35AB ET CRY3AA POUR PRÉVENIR LE DÉVELOPPEMENT DE LA RÉSISTANCE CHEZ LES CHRYSOMÈLES DES RACINES DU MAÏS (DIABROTICA SPP.)

(30) Priority: 15.04.2011 US 476005 P; 20.04.2011 US 477447 P; 30.09.2010 US 388273 P; 23.04.2010 US 327240 P
(43) Date of publication of application: 27.02.2013
(73) Proprietor: Dow AgroSciences LLC, Indianapolis, IN 46268 (US)
(72) Inventor: NARVA, Kenneth, E., Zionsville, IN 46077 (US); MEADE, Thomas, Zionsville, IN 46077 (US); FENCIL, Kristin, J., Indianapolis, IN (US); LI, Huarong, Zionsville, IN 46077 (US); HEY, Timothy, D., Zionsville, IN 46077 (US); WOOSLEY, Aaron, T., Fishers, IN 46038 (US); OLSON, Monica, Britt, Lebanon, IN 46052 (US)
(74) Representative: Krauss, Jan
(86) International application number: PCT/US2011/033617
(87) International publication number: WO 2011/133891

(56) References cited:
- US-A1- 2010 017 914
- US-B1- 6 551 962
- US-B2- 7 544 862
- KAISER-ALEXNAT ET AL: "Protease activities in the midgut of Western corn rootworm (Diabrotica virgifera virgifera LeConte)", JOURNAL OF INVERTEBRATE PATHOLOGY, SAN DIEGO, CA, US, vol. 100, no. 3, 2 March 2009 (2009-03-02) , pages 169-174, XP026075646, ISSN: 0022-2011, DOI: 10.1016/J.JIP.2009.01.003 [retrieved on 2009-01-25]
- SWAN CHRISTOPHER M ET AL: "Processing of transgenic crop residues in stream ecosystems", JOURNAL OF APPLIED ECOLOGY, vol. 46, no. 6, December 2009 (2009-12), pages 1304-1313, XP002712064,
- Kranthi K. R.: "INSECTICIDE RESISTANCE MANAGEMENT IN COTTON TO ENHANCE PRODUCTIVITY", , 15 December 2007 (2007-12-15), XP002712065, Retrieved from the Internet: URL:http://www.cicr.org.in/pdf/ELS/prot1.p df [retrieved on 2013-08-30]
- BRAVO A ET AL: "How to cope with insect resistance to Bt toxins?", TRENDS IN BIOTECHNOLOGY, ELSEVIER PUBLICATIONS, CAMBRIDGE, GB, vol. 26, no. 10, 1 October 2008 (2008-10-01), pages 573-579, XP025406825, ISSN: 0167-7799, DOI: 10.1016/J.TIBTECH.2008.06.005 [retrieved on 2008-08-14]
- LI HUARONG ET AL: "Bacillus thuringiensis Cry34Ab1/Cry35Ab1 Interactions with Western Corn Rootworm Midgut Membrane Binding Sites", PLOS ONE, vol. 8, no. 1, January 2013 (2013-01), XP002712066,
- ELLIS ET AL.: 'Novel Bacillus thuringiensis binary insecticidal crystal proteins active on western com rootworm, Diabrotica virgifera virgifera LeConte.' APPL ENVIRON MICROBIOL. vol. 68, no. 3, March 2002, pages 1137 - 45, XP002985898

### SEQUENCE LISTING

<110> DOW AGROSCIENCES LLC
<120> Combinations Including Cry34Ab/35Ab and Cry3Aa Proteins to Prevent Development of Resistance in Corn Rootworms (Diabrotica spp.)
<130> DAS-P0171-US-01
<160> 8
<170> PatentIn version 3.5
<210> 1
   <211> 644
   <212> PRT
   <213> Bacillus thuringiensis
<400> 1
   Met Asn Pro Asn Asn Arg Ser Glu His Asp Thr Ile Lys Thr Thr Glu
   1               5                   10                  15
   Asn Asn Glu Val Pro Thr Asn His Val Gln Tyr Pro Leu Ala Glu Thr
               20                  25                  30
   Pro Asn Pro Thr Leu Glu Asp Leu Asn Tyr Lys Glu Phe Leu Arg Met
           35                  40                  45
   Thr Ala Asp Asn Asn Thr Glu Ala Leu Asp Ser Ser Thr Thr Lys Asp
       50                  55                  60
   Val Ile Gln Lys Gly Ile Ser Val Val Gly Asp Leu Leu Gly Val Val
   65                  70                  75                  80
   Gly Phe Pro Phe Gly Gly Ala Leu Val Ser Phe Tyr Thr Asn Phe Leu
                   85                  90                  95
   Asn Thr Ile Trp Pro Ser Glu Asp Pro Trp Lys Ala Phe Met Glu Gln
               100                 105                 110
   Val Glu Ala Leu Met Asp Gln Lys Ile Ala Asp Tyr Ala Lys Asn Lys
           115                 120                 125
   Ala Leu Ala Glu Leu Gln Gly Leu Gln Asn Asn Val Glu Asp Tyr Val
       130                 135                 140
   Ser Ala Leu Ser Ser Trp Gln Lys Asn Pro Val Ser Ser Arg Asn Pro
   145                 150                 155                 160
   His Ser Gln Gly Arg Ile Arg Glu Leu Phe Ser Gln Ala Glu Ser His
                   165                 170                 175
   Phe Arg Asn Ser Met Pro Ser Phe Ala Ile Ser Gly Tyr Glu Val Leu
               180                 185                 190
   Phe Leu Thr Thr Tyr Ala Gln Ala Ala Asn Thr His Leu Phe Leu Leu
           195                 200                 205
   Lys Asp Ala Gln Ile Tyr Gly Glu Glu Trp Gly Tyr Glu Lys Glu Asp
       210                 215                 220
   Ile Ala Glu Phe Tyr Lys Arg Gln Leu Lys Leu Thr Gln Glu Tyr Thr
   225                 230                 235                 240
   Asp His Cys Val Lys Trp Tyr Asn Val Gly Leu Asp Lys Leu Arg Gly
                   245                 250                 255
   Ser Ser Tyr Glu Ser Trp Val Asn Phe Asn Arg Tyr Arg Arg Glu Met
               260                 265                 270
   Thr Leu Thr Val Leu Asp Leu Ile Ala Leu Phe Pro Leu Tyr Asp Val
           275                 280                 285
   Arg Leu Tyr Pro Lys Glu Val Lys Thr Glu Leu Thr Arg Asp Val Leu
       290                 295                 300
   Thr Asp Pro Ile Val Gly Val Asn Asn Leu Arg Gly Tyr Gly Thr Thr
   305                 310                 315                 320
   Phe Ser Asn Ile Glu Asn Tyr Ile Arg Lys Pro His Leu Phe Asp Tyr
                   325                 330                 335
   Leu His Arg Ile Gln Phe His Thr Arg Phe Gln Pro Gly Tyr Tyr Gly
               340                 345                 350
   Asn Asp Ser Phe Asn Tyr Trp Ser Gly Asn Tyr Val Ser Thr Arg Pro
           355                 360                 365
   Ser Ile Gly Ser Asn Asp Ile Ile Thr Ser Pro Phe Tyr Gly Asn Lys
       370                 375                 380
   Ser Ser Glu Pro Val Gln Asn Leu Glu Phe Asn Gly Glu Lys Val Tyr
   385                 390                 395                 400
   Arg Ala Val Ala Asn Thr Asn Leu Ala Val Trp Pro Ser Ala Val Tyr
                   405                 410                 415
   Ser Gly Val Thr Lys Val Glu Phe Ser Gln Tyr Asn Asp Gln Thr Asp
               420                 425                 430
   Glu Ala Ser Thr Gln Thr Tyr Asp Ser Lys Arg Asn Val Gly Ala Val
               435                 440                 445
   Ser Trp Asp Ser Ile Asp Gln Leu Pro Pro Glu Thr Thr Asp Glu Pro
       450                 455                 460
   Leu Glu Lys Gly Tyr Ser His Gln Leu Asn Tyr Val Met Cys Phe Leu
   465                 470                 475                 480
   Met Gln Gly Ser Arg Gly Thr Ile Pro Val Leu Thr Trp Thr His Lys
                   485                 490                 495
   Ser Val Asp Phe Phe Asn Met Ile Asp Ser Lys Lys Ile Thr Gln Leu
               500                 505                 510
   Pro Leu Val Lys Ala Tyr Lys Leu Gln Ser Gly Ala Ser Val Val Ala
           515                 520                 525
   Gly Pro Arg Phe Thr Gly Gly Asp Ile Ile Gln Cys Thr Glu Asn Gly
       530                 535                 540
   Ser Ala Ala Thr Ile Tyr Val Thr Pro Asp Val Ser Tyr Ser Gln Lys
   545                 550                 555                 560
   Tyr Arg Ala Arg Ile His Tyr Ala Ser Thr Ser Gln Ile Thr Phe Thr
                   565                 570                 575
   Leu Ser Leu Asp Gly Ala Pro Phe Asn Gln Tyr Tyr Phe Asp Lys Thr
               580                 585                 590
   Ile Asn Lys Gly Asp Thr Leu Thr Tyr Asn Ser Phe Asn Leu Ala Ser
           595                 600                 605
   Phe Ser Thr Pro Phe Glu Leu Ser Gly Asn Asn Leu Gln Ile Gly Val
       610                 615                 620
   Thr Gly Leu Ser Ala Gly Asp Lys Val Tyr Ile Asp Lys Ile Glu Phe
   625                 630                 635                 640
   Ile Pro Val Asn
<210> 2
   <211> 486
   <212> PRT
   <213> Bacillus thuringiensis
<400> 2
   Asn Pro His Ser Gln Gly Arg Ile Arg Glu Leu Phe Ser Gln Ala Glu
   1               5                   10                  15
   Ser His Phe Arg Asn Ser Met Pro Ser Phe Ala Ile Ser Gly Tyr Glu
               20                  25                  30
   Val Leu Phe Leu Thr Thr Tyr Ala Gln Ala Ala Asn Thr His Leu Phe
           35                  40                  45
   Leu Leu Lys Asp Ala Gln Ile Tyr Gly Glu Glu Trp Gly Tyr Glu Lys
       50                  55                  60
   Glu Asp Ile Ala Glu Phe Tyr Lys Arg Gln Leu Lys Leu Thr Gln Glu
   65                  70                  75                  80
   Tyr Thr Asp His Cys Val Lys Trp Tyr Asn Val Gly Leu Asp Lys Leu
                   85                  90                  95
   Arg Gly Ser Ser Tyr Glu Ser Trp Val Asn Phe Asn Arg Tyr Arg Arg
               100                 105                 110
   Glu Met Thr Leu Thr Val Leu Asp Leu Ile Ala Leu Phe Pro Leu Tyr
           115                 120                 125
   Asp Val Arg Leu Tyr Pro Lys Glu Val Lys Thr Glu Leu Thr Arg Asp
       130                 135                 140
   Val Leu Thr Asp Pro Ile Val Gly Val Asn Asn Leu Arg Gly Tyr Gly
   145                 150                 155                 160
   Thr Thr Phe Ser Asn Ile Glu Asn Tyr Ile Arg Lys Pro His Leu Phe
                   165                 170                 175
   Asp Tyr Leu His Arg Ile Gln Phe His Thr Arg Phe Gln Pro Gly Tyr
               180                 185                 190
   Tyr Gly Asn Asp Ser Phe Asn Tyr Trp Ser Gly Asn Tyr Val Ser Thr
           195                 200                 205
   Arg Pro Ser Ile Gly Ser Asn Asp Ile Ile Thr Ser Pro Phe Tyr Gly
       210                 215                 220
   Asn Lys Ser Ser Glu Pro Val Gln Asn Leu Glu Phe Asn Gly Glu Lys
   225                 230                 235                 240
   Val Tyr Arg Ala Val Ala Asn Thr Asn Leu Ala Val Trp Pro Ser Ala
                   245                 250                 255
   Val Tyr Ser Gly Val Thr Lys Val Glu Phe Ser Gln Tyr Asn Asp Gln
               260                 265                 270
   Thr Asp Glu Ala Ser Thr Gln Thr Tyr Asp Ser Lys Arg Asn Val Gly
           275                 280                 285
   Ala Val Ser Trp Asp Ser Ile Asp Gln Leu Pro Pro Glu Thr Thr Asp
       290                 295                 300
   Glu Pro Leu Glu Lys Gly Tyr Ser His Gln Leu Asn Tyr Val Met Cys
   305                 310                 315                 320
   Phe Leu Met Gln Gly Ser Arg Gly Thr Ile Pro Val Leu Thr Trp Thr
                   325                 330                 335
   His Lys Ser Val Asp Phe Phe Asn Met Ile Asp Ser Lys Lys Ile Thr
               340                 345                 350
   Gln Leu Pro Leu Val Lys Ala Tyr Lys Leu Gln Ser Gly Ala Ser Val
           355                 360                 365
   Val Ala Gly Pro Arg Phe Thr Gly Gly Asp Ile Ile Gln Cys Thr Glu
       370                 375                 380
   Asn Gly Ser Ala Ala Thr Ile Tyr Val Thr Pro Asp Val Ser Tyr Ser
   385                 390                 395                 400
   Gln Lys Tyr Arg Ala Arg Ile His Tyr Ala Ser Thr Ser Gln Ile Thr
                   405                 410                 415
   Phe Thr Leu Ser Leu Asp Gly Ala Pro Phe Asn Gln Tyr Tyr Phe Asp
               420                 425                 430
   Lys Thr Ile Asn Lys Gly Asp Thr Leu Thr Tyr Asn Ser Phe Asn Leu
           435                 440                 445
   Ala Ser Phe Ser Thr Pro Phe Glu Leu Ser Gly Asn Asn Leu Gln Ile
       450                 455                 460
   Gly Val Thr Gly Leu Ser Ala Gly Asp Lys Val Tyr Ile Asp Lys Ile
   465                 470                 475                 480
   Glu Phe Ile Pro Val Asn
                   485
<210> 3
   <211> 123
   <212> PRT
   <213> Bacillus thuringiensis
<400> 3
   Met Ser Ala Arg Glu Val His Ile Asp Val Asn Asn Lys Thr Gly His
   1               5                   10                  15
   Thr Leu Gln Leu Glu Asp Lys Thr Lys Leu Asp Gly Gly Arg Trp Arg
               20                  25                  30
   Thr Ser Pro Thr Asn Val Ala Asn Asp Gln Ile Lys Thr Phe Val Ala
           35                  40                  45
   Glu Ser Asn Gly Phe Met Thr Gly Thr Glu Gly Thr Ile Tyr Tyr Ser
       50                  55                  60
   Ile Asn Gly Glu Ala Glu Ile Ser Leu Tyr Phe Asp Asn Pro Phe Ala
   65                  70                  75                  80
   Gly Ser Asn Lys Tyr Asp Gly His Ser Asn Lys Ser Gln Tyr Glu Ile
                   85                  90                  95
   Ile Thr Gln Gly Gly Ser Gly Asn Gln Ser His Val Thr Tyr Thr Ile
               100                 105                 110
   Gln Thr Thr Ser Ser Arg Tyr Gly His Lys Ser
           115                 120
<210> 4
   <211> 383
   <212> PRT
   <213> Bacillus thuringiensis
<400> 4
   Met Leu Asp Thr Asn Lys Val Tyr Glu Ile Ser Asn His Ala Asn Gly
   1               5                   10                  15
   Leu Tyr Ala Ala Thr Tyr Leu Ser Leu Asp Asp Ser Gly Val Ser Leu
               20                  25                  30
   Met Asn Lys Asn Asp Asp Asp Ile Asp Asp Tyr Asn Leu Lys Trp Phe
           35                  40                  45
   Leu Phe Pro Ile Asp Asp Asp Gln Tyr Ile Ile Thr Ser Tyr Ala Ala
       50                  55                  60
   Asn Asn Cys Lys Val Trp Asn Val Asn Asn Asp Lys Ile Asn Val Ser
   65                  70                  75                  80
   Thr Tyr Ser Ser Thr Asn Ser Ile Gln Lys Trp Gln Ile Lys Ala Asn
                   85                  90                  95
   Gly Ser Ser Tyr Val Ile Gln Ser Asp Asn Gly Lys Val Leu Thr Ala
               100                 105                 110
   Gly Thr Gly Gln Ala Leu Gly Leu Ile Arg Leu Thr Asp Glu Ser Ser
           115                 120                 125
   Asn Asn Pro Asn Gln Gln Trp Asn Leu Thr Ser Val Gln Thr Ile Gln
       130                 135                 140
   Leu Pro Gln Lys Pro Ile Ile Asp Thr Lys Leu Lys Asp Tyr Pro Lys
   145                 150                 155                 160
   Tyr Ser Pro Thr Gly Asn Ile Asp Asn Gly Thr Ser Pro Gln Leu Met
                   165                 170                 175
   Gly Trp Thr Leu Val Pro Cys Ile Met Val Asn Asp Pro Asn Ile Asp
               180                 185                 190
   Lys Asn Thr Gln Ile Lys Thr Thr Pro Tyr Tyr Ile Leu Lys Lys Tyr
           195                 200                 205
   Gln Tyr Trp Gln Arg Ala Val Gly Ser Asn Val Ala Leu Arg Pro His
       210                 215                 220
   Glu Lys Lys Ser Tyr Thr Tyr Glu Trp Gly Thr Glu Ile Asp Gln Lys
   225                 230                 235                 240
   Thr Thr Ile Ile Asn Thr Leu Gly Phe Gln Ile Asn Ile Asp Ser Gly
                   245                 250                 255
   Met Lys Phe Asp Ile Pro Glu Val Gly Gly Gly Thr Asp Glu Ile Lys
               260                 265                 270
   Thr Gln Leu Asn Glu Glu Leu Lys Ile Glu Tyr Ser His Glu Thr Lys
           275                 280                 285
   Ile Met Glu Lys Tyr Gln Glu Gln Ser Glu Ile Asp Asn Pro Thr Asp
       290                 295                 300
   Gln Ser Met Asn Ser Ile Gly Phe Leu Thr Ile Thr Ser Leu Glu Leu
   305                 310                 315                 320
   Tyr Arg Tyr Asn Gly Ser Glu Ile Arg Ile Met Gln Ile Gln Thr Ser
                   325                 330                 335
   Asp Asn Asp Thr Tyr Asn Val Thr Ser Tyr Pro Asn His Gln Gln Ala
               340                 345                 350
   Leu Leu Leu Leu Thr Asn His Ser Tyr Glu Glu Val Glu Glu Ile Thr
           355                 360                 365
   Asn Ile Pro Lys Ser Thr Leu Lys Lys Leu Lys Lys Tyr Tyr Phe
   370 375 380
<210> 5
   <211> 354
   <212> PRT
   <213> Bacillus thuringiensis
<400> 5
   Met Leu Asp Thr Asn Lys Val Tyr Glu Ile Ser Asn His Ala Asn Gly
   1               5                   10                  15
   Leu Tyr Ala Ala Thr Tyr Leu Ser Leu Asp Asp Ser Gly Val Ser Leu
               20                  25                  30
   Met Asn Lys Asn Asp Asp Asp Ile Asp Asp Tyr Asn Leu Lys Trp Phe
           35                  40                  45
   Leu Phe Pro Ile Asp Asp Asp Gln Tyr Ile Ile Thr Ser Tyr Ala Ala
       50                  55                  60
   Asn Asn Cys Lys Val Trp Asn Val Asn Asn Asp Lys Ile Asn Val Ser
   65                  70                  75                  80
   Thr Tyr Ser Ser Thr Asn Ser Ile Gln Lys Trp Gln Ile Lys Ala Asn
                   85                  90                  95
   Gly Ser Ser Tyr Val Ile Gln Ser Asp Asn Gly Lys Val Leu Thr Ala
               100                 105                 110
   Gly Thr Gly Gln Ala Leu Gly Leu Ile Arg Leu Thr Asp Glu Ser Ser
           115                 120                 125
   Asn Asn Pro Asn Gln Gln Trp Asn Leu Thr Ser Val Gln Thr Ile Gln
       130                 135                 140
   Leu Pro Gln Lys Pro Ile Ile Asp Thr Lys Leu Lys Asp Tyr Pro Lys
   145                 150                 155                 160
   Tyr Ser Pro Thr Gly Asn Ile Asp Asn Gly Thr Ser Pro Gln Leu Met
                   165                 170                 175
   Gly Trp Thr Leu Val Pro Cys Ile Met Val Asn Asp Pro Asn Ile Asp
               180                 185                 190
   Lys Asn Thr Gln Ile Lys Thr Thr Pro Tyr Tyr Ile Leu Lys Lys Tyr
           195                 200                 205
   Gln Tyr Trp Gln Arg Ala Val Gly Ser Asn Val Ala Leu Arg Pro His
       210                 215                 220
   Glu Lys Lys Ser Tyr Thr Tyr Glu Trp Gly Thr Glu Ile Asp Gln Lys
   225                 230                 235                 240
   Thr Thr Ile Ile Asn Thr Leu Gly Phe Gln Ile Asn Ile Asp Ser Gly
                   245                 250                 255
   Met Lys Phe Asp Ile Pro Glu Val Gly Gly Gly Thr Asp Glu Ile Lys
               260                 265                 270
   Thr Gln Leu Asn Glu Glu Leu Lys Ile Glu Tyr Ser His Glu Thr Lys
           275                 280                 285
   Ile Met Glu Lys Tyr Gln Glu Gln Ser Glu Ile Asp Asn Pro Thr Asp
       290                 295                 300
   Gln Ser Met Asn Ser Ile Gly Phe Leu Thr Ile Thr Ser Leu Glu Leu
   305                 310                 315                 320
   Tyr Arg Tyr Asn Gly Ser Glu Ile Arg Ile Met Gln Ile Gln Thr Ser
                   325                 330                 335
   Asp Asn Asp Thr Tyr Asn Val Thr Ser Tyr Pro Asn His Gln Gln Ala
               340                 345                 350
   Leu Leu
<210> 6
   <211> 430
   <212> DNA
   <213> Bacillus thuringiensis
<400> 6
   aatctactag taaaaaggag ataaccatgt cagcacgtga agtacacatt gatgtaaata      60
   ataagacagg tcatacatta caattagaag ataaaacaaa acttgatggt ggtagatggc     120
   gaacatcacc tacaaatgtt gctaatgatc aaattaaaac atttgtagca gaatcaaatg     180
   gttttatgac aggtacagaa ggtactatat attatagcat aaatggagaa gcagaaatta     240
   gtttatattt tgacaatcct tttgcaggtt ctaataaata tgatggacat tccaataaat     300
   ctcaatatga aattattacc caaggaggat caggaaatca atctcatgtt acgtatacta     360
   ttcaaaccac atcctcacga tatgggcata aatcataagg aaaacgggta ccgtcgacct     420
   cgagcccaaa                                                            430
<210> 7
   <211> 1062
   <212> DNA
   <213> Bacillus thuringiensis
<400> 7
   atgctggaca ccaacaaagt ctacgagatt tccaaccacg cgaacggact ttacgcagct      60
   acatatcttt cgctcgatga ctcgggtgtg tctctgatga acaagaatga tgacgacatt     120
   gatgactaca acctcaagtg gtttctcttc cccattgatg atgaccagta catcattact     180
   tcttatgctg ccaacaactg caaagtttgg aatgtgaaca atgataagat caatgtcagc     240
   acctacagct cgaccaattc gatccagaag tggcagatca aagccaatgg ttcaagctac     300
   gttatccaat ccgacaatgg caaggtcctc acagctggca ctggccaagc cctcggtctg     360
   atcagactga ccgatgagtc ctccaacaac ccaaaccagc aatggaacct tacttccgtc     420
   caaacaatcc agctgccaca gaagcccatc attgacacaa agctgaaaga ctatcctaag     480
   tactcaccta ctggcaacat tgacaatggg acgtctcccc agctgatggg ctggacgctg     540
   gtgccgtgta tcatggtcaa tgacccaaac atcgacaaga acactcagat caagaccact     600
   ccttactaca tcctcaagaa gtatcagtac tggcagagag cggttggcag caatgtggca     660
   ctcagacccc atgagaagaa gtcatacacc tatgaatggg gaaccgaaat cgatcagaaa     720
   acgaccatca tcaacacgct cgggttccag atcaacatag actcgggaat gaagtttgac     780
   atcccagagg tgggaggtgg cacagatgag atcaagaccc agttgaatga ggaactgaag     840
   attgagtaca gccatgagac gaagatcatg gagaagtatc aagaacaaag cgagatcgac     900
   aacccgacag atcagagcat gaactcaata gggttcttga ccattactag tttggagctt     960
   tacagataca atggctcaga gataaggatc atgcagattc agacgtccga caatgacacc    1020
   tacaacgtga cgagctatcc gaaccaccag caagccttgc tg                       1062
<210> 8
   <211> 2441
   <212> DNA
   <213> Bacillus thuringiensis
<400> 8
   ctactagtag gagatttcca tgaatccgaa caatcgaagt gaacatgata caataaaaac 60
   tactgaaaat aatgaggtgc caactaacca tgttcaatat cctttagcgg aaactccaaa 120
   tccaacacta gaagatttaa attataaaga gtttttaaga atgactgcag ataataatac 180
   ggaagcacta gatagctcta caacaaaaga tgtcattcaa aaaggcattt ccgtagtagg 240
   tgatctccta ggcgtagtag gtttcccgtt tggtggagcg cttgtttcgt tttatacaaa 300
   ctttttaaat actatttggc caagtgaaga cccgtggaag gcttttatgg aacaagtaga 360
   agcattgatg gatcagaaaa tagctgatta tgcaaaaaat aaagctcttg cagagttaca 420
   gggccttcaa aataatgtcg aagattatgt gagtgcattg agttcatggc aaaaaaatcc 480
   tgtgagttca cgaaatccac atagccaggg gcggataaga gagctgtttt ctcaagcaga 540
   aagtcatttt cgtaattcaa tgccttcgtt tgcaatttct ggatacgagg ttctatttct 600
   aacaacatat gcacaagctg ccaacacaca tttattttta ctaaaagacg ctcaaattta 660
   tggagaagaa tggggatacg aaaaagaaga tattgctgaa ttttataaaa gacaactaaa 720
   acttacgcaa gaatatactg accattgtgt caaatggtat aatgttggat tagataaatt 780
   aagaggttca tcttatgaat cttgggtaaa ctttaaccgt tatcgcagag agatgacatt 840
   aacagtatta gatttaattg cactatttcc attgtatgat gttcggctat acccaaaaga 900
   agttaaaacc gaattaacaa gagacgtttt aacagatcca attgtcggag tcaacaacct 960
   taggggctat ggaacaacct tctctaatat agaaaattat attcgaaaac cacatctatt 1020
   tgactatctg catagaattc aatttcacac gcggttccaa ccaggatatt atggaaatga 1080
   ctctttcaat tattggtccg gtaattatgt ttcaactaga ccaagcatag gatcaaatga 1140
   tataatcaca tctccattct atggaaataa atccagtgaa cctgtacaaa atttagaatt 1200
   taatggagaa aaagtctata gagccgtagc aaatacaaat cttgcggtct ggccgtccgc 1260
   tgtatattca ggtgttacaa aagtggaatt tagccaatat aatgatcaaa cagatgaagc 1320
   aagtacacaa acgtacgact caaaaagaaa tgttggcgcg gtcagctggg attctatcga 1380
   tcaattgcct ccagaaacaa cagatgaacc tctagaaaag ggatatagcc atcaactcaa 1440
   ttatgtaatg tgctttttaa tgcagggtag tagaggaaca atcccagtgt taacttggac 1500
   acataaaagt gtagactttt ttaacatgat tgattcgaaa aaaattacac aacttccgtt 1560
   agtaaaggca tataagttac aatctggtgc ttccgttgtc gcaggtccta ggtttacagg 1620
   aggagatatc attcaatgca cagaaaatgg aagtgcggca actatttacg ttacaccgga 1680
   tgtgtcgtac tctcaaaaat atcgagctag aattcattat gcttctacat ctcagataac 1740
   atttacactc agtttagacg gggcaccatt taatcaatac tatttcgata aaacgataaa 1800
   taaaggagac acattaacgt ataattcatt taatttagca agtttcagca caccattcga 1860
   attatcaggg aataacttac aaataggcgt cacaggatta agtgctggag ataaagttta 1920
   tatagacaaa attgaattta ttccagtgaa ttaaattaac tagaaagtaa agaagtagtg 1980
   accatctatg atagtaagca aaggataaaa aaatgagttc ataaaatgaa taacatagtg 2040
   ttcttcaact ttcgcttttt gaaggtagat gaagaacact atttttattt tcaaaatgaa 2100
   ggaagtttta aatatgtaat catttaaagg gaacaatgaa agtaggaaat aagtcattat 2160
   ctataacaaa ataacatttt tatatagcca gaaatgaatt ataatattaa tcttttctaa 2220
   attgacgttt ttctaaacgt tctatagctt caagacgctt agaatcatca atatttgtat 2280
   acagagctgt tgtttccatc gagttatgtc ccatttgatt cgctaataga acaagatctt 2340
   tattttcgtt ataatgattg gttgcataag tatggcgtaa tttatgaggg cttttctttt 2400
   catcaaaagc cctcgtgtat ttctctgtaa gcttcgggct g 2441

## Description

### BACKGROUND

Humans grow corn for food and energy applications. Corn is an important crop. It is an important source of food, food products, and animal feed in many areas of the world. Insects eat and damage plants and thereby undermine these human efforts. Billions of dollars are spent each year to control insect pests and additional billions are lost to the damage they inflict.

Damage caused by insect pests is a major factor in the loss of the world's corn crops, despite the use of protective measures such as chemical pesticides. In view of this, insect resistance has been genetically engineered into crops such as corn in order to control insect damage and to reduce the need for traditional chemical pesticides.

Over 10 million acres of U.S. corn are infested with corn rootworm species complex each year. The corn rootworm species complex includes the northern corn rootworm (Diabrotica barberi), the southern corn rootworm (D. undecimpunctata howardi), and the western corn rootworm (D. virgifera virgifera). (Other species include Diabrotica virgifera zeae (Mexican corn rootworm), Diabrotica balteata (Brazilian corn rootworm), and Brazilian corn rootworm complex (Diabrotica viridula and Diabrotica speciosa).)

The soil-dwelling larvae of these Diabrotica species feed on the root of the corn plant, causing lodging. Lodging eventually reduces corn yield and often results in death of the plant. By feeding on cornsilks, the adult beetles reduce pollination and, therefore, detrimentally affect the yield of corn per plant. In addition, adults and larvae of the genus Diabrotica attack cucurbit crops (cucumbers, melons, squash, etc.) and many vegetable and field crops in commercial production as well as those being grown in home gardens.

Synthetic organic chemical insecticides have been the primary tools used to control insect pests but biological insecticides, such as the insecticidal proteins derived from *Bacillus thuringiensis* (*Bt*), have played an important role in some areas. The ability to produce insect-resistant plants through transformation with *Bt* insecticidal protein genes has revolutionized modem agriculture and heightened the importance and value of insecticidal proteins and their genes.

Insecticidal crystal proteins from some strains of Bacillus thuringiensis (B.t.) are well-known in the art. See, e.g., Hofte et al., Microbial Reviews, Vol. 53, No. 2, pp. 242-255 (1989). These proteins are typically produced by the bacteria as approximately 130 kDa protoxins that are then cleaved by proteases in the insect midgut, after ingestion by the insect, to yield a roughly 60 kDa core toxin. These proteins are known as crystal proteins because distinct crystalline inclusions can be observed with spores in some strains of B.t. These crystalline inclusions are often composed of several distinct proteins.

One group of genes which have been utilized for the production of transgenic insect resistant crops are the delta-endotoxins from Bacillus thuringiensis (B.t.). Delta-endotoxins have been successfully expressed in crop plants such as cotton, potatoes, rice, sunflower, as well as corn, and have proven to provide excellent control over insect pests. (Perlak, F. J et al. (1990) Bio/Technology 8, 939-943; Perlak, F. J. et al. (1993) Plant Mol. Biol. 22: 313-321; Fujimoto H. et al. (1993) Bio/Technology 11: 1151-1155; Tu et al. (2000) Nature Biotechnology 18:1101-1104; PCT publication number WO 01/13731; and Bing J W et al. (2000) Efficacy of Cry IF Transgenic Maize, 14th Biennial International Plant Resistance to Insects Workshop, Fort Collins, Colo.)

Several *Bt* proteins have been used to create the insect-resistant transgenic plants that have been successfully registered and commercialized to date. These include Cry1Ab, Cry1Ac, Cry1F, Cry3Aa, and Cry3Bb in corn, Cry1Ac and Cry2Ab in cotton, and Cry3A in potato. There is also SMART STAX in corn, which comprises Cry1A.105 and Cry2Ab.

The commercial products expressing these proteins express a single protein except in cases where the combined insecticidal spectrum of 2 proteins is desired (*e.g*., Cry1Ab and Cry3Bb in corn combined to provide resistance to lepidopteran pests and rootworm, respectively) or where the independent action of the proteins makes them useful as a tool for delaying the development of resistance in susceptible insect populations (*e.g*., Cry1Ac and Cry2Ab in cotton combined to provide resistance management for tobacco budworm).

Some of the qualities of insect-resistant transgenic plants that have led to rapid and widespread adoption of this technology also give rise to the concern that pest populations will develop resistance to the insecticidal proteins produced by these plants. Several strategies have been suggested for preserving the utility of *Bt*-based insect resistance traits which include deploying proteins at a high dose in combination with a refuge, and alternation with, or co-deployment of, different toxins (McGaughey et al. (1998), "B.t. Resistance Management," Nature Biotechnol. 16:144-146).

The proteins selected for use in an Insect Resistance Management (IRM) stack should be active such that resistance developed to one protein does not confer resistance to the second protein (*i.e.,* there is not cross resistance to the proteins). If, for example, a pest population selected for resistance to "Protein A" is sensitive to "Protein B", one would conclude that there is not cross resistance and that a combination of Protein A and Protein B would be effective in delaying resistance to Protein A alone.

In the absence of resistant insect populations, assessments can be made based on other characteristics presumed to be related to cross-resistance potential. The utility of receptor-mediated binding in identifying insecticidal proteins likely to not exhibit cross resistance has been suggested (van Mellaert *et al.* 1999). The key predictor of lack of cross resistance inherent in this approach is that the insecticidal proteins do not compete for receptors in a sensitive insect species.

In the event that two *Bt* toxins compete for the same receptor, then if that receptor mutates in that insect so that one of the toxins no longer binds to that receptor and thus is no longer insecticidal against the insect, it might be the case that the insect will also be resistant to the second toxin (which competitively bound to the same receptor). That is, the insect is said to be cross-resistant to both *Bt* toxins. However, if two toxins bind to two different receptors, this could be an indication that the insect would not be simultaneously resistant to those two toxins.

A relatively newer insecticidal protein system was discovered in Bacillus thuringiensis as disclosed in WO 97/40162. This system comprises two proteins -- one of approximately 15 kDa and the other of about 45 kDa. See also U.S. Pat. Nos. 6,083,499 and 6,127,180. These proteins have now been assigned to their own classes, and accordingly received the Cry designations of Cry34 and Cry35, respectively. See Crickmore et al. website (biols.susx.ac.uk/home/Neil_Crickmore/Bt/). Many other related proteins of this type of system have now been disclosed. See e.g. U.S. Pat. No. 6,372,480; WO 01/14417; and WO 00/66742. Plant-optimized genes that encode such proteins, wherein the genes are engineered to use codons for optimized expression in plants, have also been disclosed. See e.g. U.S. Pat. No. 6,218,188.

The exact mode of action of the Cry34/35 system has yet to be determined, but it appears to form pores in membranes of insect gut cells. See Moellenbeck et al., Nature Biotechnology, vol. 19, p. 668 (July 2001); Masson et al., Biochemistry, 43 (12349-12357) (2004). The exact mechanism of action remains unclear despite 3D atomic coordinates and crystal structures being known for a Cry34 and a Cry35 protein. *See* U.S. Patent Nos. 7,524,810 and 7,309,785. For example, it is unclear if one or both of these proteins bind a typical type of receptor, such as an alkaline phosphatase or an aminopeptidase. Furthermore, because there are different mechanisms by which an insect can develop resistance to a Cry protein (such as by altered glycosylation of the receptor [see Jurat-Fuentes et al. (2002) 68 AEM 5711-5717], by removal of the receptor protein [see Lee et al. (1995) 61 AEM 3836-3842], by mutating the receptor or by other mechanisms [see Heckel et al., J. Inv. Pathol. 95 (2007) 192-197]), it was impossible to *a priori* predict whether there would be cross-resistance between Cry34/35 and other Cry proteins. Predicting competitive binding for the Cry34/35 system is also further complicated by the fact that two proteins are involved in the Cry34/35 binary system. Again, it is unclear if and how these proteins effectively bind the insect gut / gut cells, and if and how they interact with or bind with each other.

Other options for controlling coleopterans include Cry3Bb toxins, Cry3C, Cry6B, ET29, ET33 with ET34, TIC407, TIC435, TIC417, TIC901, TIC1201, ET29 with TIC810, ET70, ET76 with ET80, TIC851, and others. RNAi approaches have also been proposed. *See e.g.* Baum et al., Nature Biotechnology, vol. 25, no. 11 (Nov. 2007) pp. 1322-1326.

### BRIEF SUMMARY

The subject invention relates in part to Cry34Ab/35Ab in combination with Cry3Aa. The subject invention relates in part to the surprising discovery that Cry34Ab/Cry35Ab and Cry3Aa are useful for preventing development of resistance (to either insecticidal protein system alone) by a corn rootworm (*Diabrotica* spp.) population. As one skilled in the art will recognize with the benefit of this disclosure, plants producing these insecticidal Cry proteins will be useful to mitigate concern that a corn rootworm population could develop that would be resistant to either of these insecticidal protein systems alone.

The subject invention is supported in part by the discovery that components of these Cry protein systems do not compete with each other for binding corn rootworm gut receptors.

The subject invention also relates in part to triple stacks or "pyramids" of three (or more) toxin systems, with Cry34Ab/Cry35Ab and Cry3Aa being the base pair. Thus, plants (and acreage planted with such plants) that produce these two insecticidal protein systems are included within the scope of the subject invention.

### BRIEF DESCRIPTION OF THE FIGURES

The detailed description of the drawings particularly refers to the accompanying figures in which:

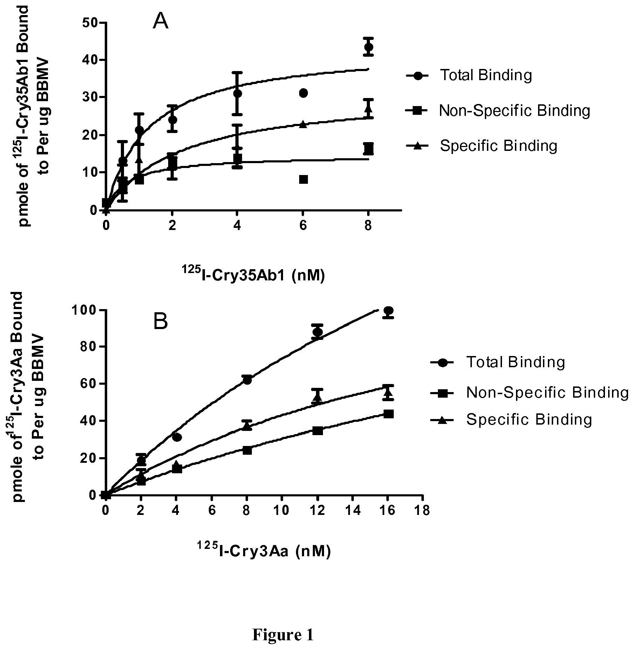

**Figure 1****.** Binding of ¹²⁵I-Cry35Ab1 (**A**) and ¹²⁵I-Cry3Aa (**B**) as a function of input radiolabeled Cry toxins to BBMV prepared from western corn rootworm larvae. Specific binding =total binding - non-specific binding, error bar=SEM (standard error of mean).
   Binding experiments demonstrated that both ¹²⁵I-Cry35Ab1 and ¹²⁵I-Cry3Aa were able to specifically bind to the BBMV (Figure 1A and 1B). ¹²⁵I-Cry35Ab1 and ¹²⁵I-Cry3Aa had a binding affinity Kd=2.31±1.26, 24.0±9.7 (nM), respectively, and a binding site concentration Bmax=31.69±6.38, 146.3±39.9 (pmole/ug BBMV), respectively.

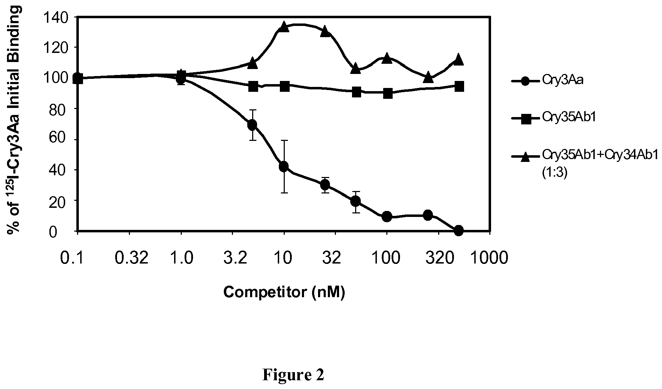

**Figure 2****.** Binding of ¹²⁵I-Cry3Aa to BBMV prepared from western corn rootworm larvae at different concentrations of non-labeled competitor. Error bars for the homologous competition binding represent standard deviation, and the error bars for heterologous competition are not shown.
   Experimental results demonstrated that Cry3Aa was able to completely displace itself when the molar concentration increased to 500 nM (100 folds excess ¹²⁵I-Cry3Aa) (Figure 2). However, either Cry35Ab1 alone or the mixture of Cry35Ab1+Cry34Ab1 (1:3) was not able to displace ¹²⁵I-Cry3Aa. These data indicated that Cry35Ab1 alone or Cry35Ab1+Cry34Ab1 (1:3) does not share a receptor with Cry3Aa.

### BRIEF DESCRIPTION OF THE SEQUENCES

SEQ ID NO:1: Full length, native Cry3Aa protein sequence
SEQ ID NO:2: Trypsin-truncated Cry3Aa core protein sequence
SEQ ID NO:3: Full length, native Cry34Ab1 protein sequence
SEQ ID NO:4: Full length, native Cry35Ab1 protein sequence
SEQ ID NO:5: Chymotrypsin-truncated Cry35Ab1 core protein sequence
SEQ ID NO:6: cry34Ab DNA
SEQ ID NO:7: cry35Ab DNA
SEQ ID NO:8: cry3Aa DNA

### DETAILED DESCRIPTION

Sequences for the Cry34Ab/35Ab protein are obtainable from *Bacillus thruingiensis* isolate PS149B1, for example. For other genes, protein sequences, and source isolates for use according to the subject invention, see the Crickmore et al. website (lifesci.sussex.ac.uk/home/Neil_Crickmore/ Bt/intro.html), for example.

The subject invention includes the use of Cry34Ab/35Ab insecticidal proteins in combination with a Cry3Aa toxin to protect corn from damage and yield loss caused by corn rootworm feeding by corn rootworm populations that might develop resistance to either of these Cry protein systems alone (without the other).

The subject invention thus teaches an Insect Resistance Management (IRM) stack to prevent the development of resistance by corn rootworm to Cry3Aa and/or Cry34Ab/35Ab.

The present disclosure provides compositions for controlling rootworm pests comprising cells that produce a Cry3Aa toxin protein and a Cry34Ab/35Ab toxin system.

The invention further comprises a host transformed to produce both a Cry3Aa protein and a Cry34Ab/35Ab binary toxin, wherein said host is a plant cell.

It is additionally intended that the invention provides a method of controlling rootworm pests comprising contacting said pests or the environment of said pests with an effective amount of a composition that contains a Cry3Aa protein and further contains a Cry34Ab/35Ab binary toxin.

An embodiment of the invention comprises a maize plant comprising a plant-expressible gene encoding a Cry34Ab/35Ab binary toxin and a plant-expressible gene encoding a Cry3Aa protein, and seed of such a plant.

A further disclosure comprises a maize plant wherein a plant-expressible gene encoding a Cry34Ab/35Ab binary toxin and a plant-expressible gene encoding a Cry3Aa protein have been introgressed into said maize plant, and seed of such a plant.

As described in the Examples, competitive receptor binding studies using radiolabeled Cry35Ab core toxin protein show that the Cry3Aa core toxin protein does not compete for binding in CRW insect tissue samples to which Cry35Ab binds. *See* **Figure 2****.** These results indicate that the combination of Cry3Aa and Cry34Ab/35Ab proteins is an effective means to mitigate the development of resistance in CRW populations to either protein system alone.

Thus, based in part on the data described above and elsewhere herein, Cry34Ab/35Ab and Cry3Aa proteins can be used to produce IRM combinations for prevention or mitigation of resistance development by CRW. Other proteins can be added to this combination to expand insect-control spectrum, for example. The subject pair/combination can also be used in some preferred "triple stacks" or "pyramid" in combination with yet another protein for controlling rootworms, such as Cry3Ba and/or Cry6Aa. RNAi against rootworms is a still further option. *See e.g.* Baum et al., Nature Biotechnology, vol. 25, no. 11 (Nov. 2007) pp. 1322-1326. Furthermore, in light of the data and teachings presented herein, one could substitute Cry3Ba and/or Cry6Aa in place of the Cry3Aa exemplified herein as the base combination pairing with Cry34A/35A. Thus, the subject combinations provide multiple modes of action against a rootworm.

Deployment options of the subject invention include the use of Cry3Aa and Cry34Ab/35Ab proteins in corn-growing regions where (*Diabrotica* spp. are problematic. Another deployment option would be to use one or both of the Cry3Aa and Cry34Ab/35Ab proteins in combination with other traits.

In light of the disclosure of USSN 61/388,273 (filed September 30, 2010) relating to combinations of Cry34Ab/35Ab and Cry6Aa proteins, USSN 61/476,005 (filed April 15, 2011) relating to combinations of Cry34Ab/35Ab and Cry3Ba proteins, and USSN 61/477,447 (filed April 20, 2011) relating to combinations of Cry3Aa and Cry6Aa, some preferred "triple stacks" or "multiple modes of action stacks" of the subject invention include a Cry3Aa protein combined with Cry34Ab/35Ab proteins, together with a Cry6Aa protein and/or a Cry3Ba protein. Transgenic plants, including corn, comprising a cry3Ba gene, cry34Ab/35Ab genes, and a third or fourth toxin system (e.g., cry3Aa and/or cry6Aa gene(s)) are included within the scope of the subject invention. Thus, such embodiments target the insect with at least three modes of action.

A person skilled in this art will appreciate that *Bt* toxins, even within a certain class such as Cry3Aa and Cry34Ab/35Ab can vary to some extent.

Genes and toxins. The term "isolated" refers to a polynucleotide in a non-naturally occurring construct, or to a protein in a purified or otherwise non-naturally occurring state. The genes and toxins useful according to the subject invention include not only the full length sequences disclosed but also fragments of these sequences, variants, mutants, and fusion proteins which retain the characteristic pesticidal activity of the toxins specifically exemplified herein. As used herein, the terms "variants" or "variations" of genes refer to nucleotide sequences which encode the same toxins or which encode equivalent toxins having pesticidal activity. As used herein, the term "equivalent toxins" refers to toxins having the same or essentially the same biological activity against the target pests as the claimed toxins. Domains/subdomains of these proteins can be swapped to make chimeric proteins. *See e.g.* U.S. Patent No. 7,309,785 and 7,524,810. The '785 patent also teaches truncated Cry35 proteins. Truncated toxins are also exemplified herein.

As used herein, the boundaries represent approximately 95% (Cry3Aa's and Cry34Ab's and Cry35Ab's), 78% (Cry3A's and Cry 34A's and Cry35A's), and 45% (Cry3's and Cry 34's and Cry 35's) sequence identity, per "Revision of the Nomenclature for the Bacillus thuringiensis Pesticidal Crystal Proteins," N. Crickmore, D.R. Zeigler, J. Feitelson, E. Schnepf, J. Van Rie, D. Lereclus, J. Baum, and D.H. Dean. Microbiology and Molecular Biology Reviews (1998) Vol 62: 807-813. The same applies to Cry6's if used in triple stacks, for example, according to the subject invention.

It should be apparent to a person skilled in this art that genes encoding active toxins can be identified and obtained through several means. The specific genes or gene portions exemplified herein may be obtained from the isolates deposited at a culture depository. These genes, or portions or variants thereof, may also be constructed synthetically, for example, by use of a gene synthesizer. Variations of genes may be readily constructed using standard techniques for making point mutations. Also, fragments of these genes can be made using commercially available exonucleases or endonucleases according to standard procedures. For example, enzymes such as Bal31 or site-directed mutagenesis can be used to systematically cut off nucleotides from the ends of these genes. Genes that encode active fragments may also be obtained using a variety of restriction enzymes. Proteases may be used to directly obtain active fragments of these protein toxins.

Fragments and equivalents which retain the pesticidal activity of the exemplified toxins would be within the disclosure. Also, because of the redundancy of the genetic code, a variety of different DNA sequences can encode the amino acid sequences disclosed herein. It is well within the skill of a person trained in the art to create these alternative DNA sequences encoding the same, or essentially the same, toxins. These variant DNA sequences disclosed. As used herein, reference to "essentially the same" sequence refers to sequences which have amino acid substitutions, deletions, additions, or insertions which do not materially affect pesticidal activity. Fragments of genes encoding proteins that retain pesticidal activity are also included in this definition.

A further method for identifying the genes encoding the toxins and gene portions useful according to the subject invention is through the use of oligonucleotide probes. These probes are detectable nucleotide sequences. These sequences may be detectable by virtue of an appropriate label or may be made inherently fluorescent as described in International Application No. WO93/16094. As is well known in the art, if the probe molecule and nucleic acid sample hybridize by forming a strong bond between the two molecules, it can be reasonably assumed that the probe and sample have substantial homology. Preferably, hybridization is conducted under stringent conditions by techniques well-known in the art, as described, for example, in Keller, G. H., M. M. Manak (1987) DNA Probes, Stockton Press, New York, N.Y., pp. 169-170. Some examples of salt concentrations and temperature combinations are as follows (in order of increasing stringency): 2X SSPE or SSC at room temperature; 1X SSPE or SSC at 42° C; 0.1X SSPE or SSC at 42° C; 0.1X SSPE or SSC at 65° C. Detection of the probe provides a means for determining in a known manner whether hybridization has occurred. Such a probe analysis provides a rapid method for identifying toxin-encoding genes of the subject invention. The nucleotide segments which are used as probes according to the invention can be synthesized using a DNA synthesizer and standard procedures. These nucleotide sequences can also be used as PCR primers to amplify genes of the subject invention.

Variant toxins. Certain toxins of the subject invention have been specifically exemplified herein. Since these toxins are merely exemplary of the toxins of the subject invention, it should be readily apparent that the subject invention comprises variant or equivalent toxins (and nucleotide sequences coding for equivalent toxins) having the same or similar pesticidal activity of the exemplified toxin. Equivalent toxins will have amino acid homology with an exemplified toxin. This amino acid identity will typically be greater than 75%, or preferably greater than 85%, preferably greater than 90%, preferably greater than 95%, preferably greater than 96%, preferably greater than 97%, preferably greater than 98%, or preferably greater than 99% in some embodiments. The amino acid identity will typically be highest in critical regions of the toxin which account for biological activity or are involved in the determination of three-dimensional configuration which ultimately is responsible for the biological activity. In this regard, certain amino acid substitutions are acceptable and can be expected if these substitutions are in regions which are not critical to activity or are conservative amino acid substitutions which do not affect the three-dimensional configuration of the molecule. For example, amino acids may be placed in the following classes: non-polar, uncharged polar, basic, and acidic. Conservative substitutions whereby an amino acid of one class is replaced with another amino acid of the same type fall within the scope of the subject invention so long as the substitution does not materially alter the biological activity of the compound. Table 1 provides a listing of examples of amino acids belonging to each class.

**TABLE 1**

| **Class of Amino Acid** | **Examples of Amino Acids** |
|---|---|
| Nonpolar | Ala, Val, Leu, Ile, Pro, Met, Phe, Trp |
| Uncharged Polar | Gly, Ser, Thr, Cys, Tyr, Asn, Gln |
| Acidic | Asp, Glu |
| Basic | Lys, Arg, His |

In some instances, non-conservative substitutions can also be made. The critical factor is that these substitutions must not significantly detract from the biological activity of the toxin.

Recombinant hosts. The genes encoding the toxins of the subject invention can be introduced into a wide variety of microbial or plant hosts. Expression of the toxin gene results, directly or indirectly, in the intracellular production and maintenance of the pesticide.

Conjugal transfer and recombinant transfer can be used to create a *Bt* strain that expresses both toxins of the subject invention. Other host organisms may also be transformed with one or both of the toxin genes then used to accomplish the synergistic effect. With suitable microbial hosts, *e.g., Pseudomonas,* the microbes can be applied to the situs of the pest, where they will proliferate and be ingested. The result is control of the pest. Alternatively, the microbe hosting the toxin gene can be treated under conditions that prolong the activity of the toxin and stabilize the cell. The treated cell, which retains the toxic activity, then can be applied to the environment of the target pest. Non-regenerable / non-totipotent plant cells from a plant of the subject invention (comprising at least one of the subject IRM genes) are included within the subject invention.

Plant transformation. A preferred embodiment of the subject invention is the transformation of plants with genes encoding the subject insecticidal protein or its variants. The transformed plants are resistant to attack by an insect target pest by virtue of the presence of controlling amounts of the subject insecticidal protein or its variants in the cells of the transformed plant. By incorporating genetic material that encodes the insecticidal properties of the *B.t*. insecticidal toxins into the genome of a plant eaten by a particular insect pest, the adult or larvae would die after consuming the food plant. Numerous members of the monocotyledonous and dicotyledonous classifications have been transformed. Transgenic agronomic crops as well as fruits and vegetables are of commercial interest. Such crops include, but are not limited to, maize, rice, soybeans, canola, sunflower, alfalfa, sorghum, wheat, cotton, peanuts, tomatoes, potatoes, and the like. Several techniques exist for introducing foreign genetic material into plant cells, and for obtaining plants that stably maintain and express the introduced gene. Such techniques include acceleration of genetic material coated onto microparticles directly into cells (US Patent No. 4945050 and US Patent No. 5141131). Plants may be transformed using *Agrobacterium* technology, see US Patent No. 5177010, US Patent No. 5104310, European Patent Application No. 0131624B1, European Patent Application No. 120516, European Patent Application No. 159418B1, European Patent Application No. 176112, US Patent No. 5149645, US Patent No. 5469976, US Patent No. 5464763, US Patent No. 4940838, US Patent No. 4693976, European Patent Application No. 116718, European Patent Application No. 290799, European Patent Application No. 320500, European Patent Application No. 604662, European Patent Application No. 627752, European Patent Application No. 0267159, European Patent Application No. 0292435, US Patent No. 5231019, US Patent No. 5463174, US Patent No. 4762785, US Patent No. 5004863, and US Patent No. 5159135. Other transformation technology includes WHISKERS™ technology, see US Patent No. 5302523 and US Patent No. 5464765. Electroporation technology has also been used to transform plants, see WO 87/06614, US Patent No. 5472869, US Patent No. 5384253, WO 9209696, and WO 9321335. In addition to numerous technologies for transforming plants, the type of tissue which is contacted with the foreign genes may vary as well. Such tissue would include but would not be limited to embryogenic tissue, callus tissue types I and II, hypocotyl, meristem, and the like. Almost all plant tissues may be transformed during dedifferentiation using appropriate techniques within the skill of an artisan.

Genes encoding any of the subject toxins can be inserted into plant cells using a variety of techniques which are well known in the art as disclosed above. For example, a large number of cloning vectors comprising a marker that permits selection of the transformed microbial cells and a replication system functional in *Escherichia coli* are available for preparation and modification of foreign genes for insertion into higher plants. Such manipulations may include, for example, the insertion of mutations, truncations, additions, or substitutions as desired for the intended use. The vectors comprise, for example, pBR322, pUC series, M13mp series, pACYC184, *etc.* Accordingly, the sequence encoding the Cry protein or variants can be inserted into the vector at a suitable restriction site. The resulting plasmid is used for transformation of cells of *E. coli,* the cells of which are cultivated in a suitable nutrient medium, then harvested and lysed so that workable quantities of the plasmid are recovered. Sequence analysis, restriction fragment analysis, electrophoresis, and other biochemical-molecular biological methods are generally carried out as methods of analysis. After each manipulation, the DNA sequence used can be cleaved and joined to the next DNA sequence. Each manipulated DNA sequence can be cloned in the same or other plasmids.

The use of T-DNA-containing vectors for the transformation of plant cells has been intensively researched and sufficiently described in EP 120516; Lee and Gelvin (2008), Fraley *et al.* (1986), and An *et al.* (1985), and is well established in the field.

Once the inserted DNA has been integrated into the plant genome, it is relatively stable throughout subsequent generations. The vector used to transform the plant cell normally contains a selectable marker gene encoding a protein that confers on the transformed plant cells resistance to a herbicide or an antibiotic, such as bialaphos, kanamycin, G418, bleomycin, or hygromycin, *inter alia.* The individually employed selectable marker gene should accordingly permit the selection of transformed cells while the growth of cells that do not contain the inserted DNA is suppressed by the selective compound.

A large number of techniques are available for inserting DNA into a host plant cell. Those techniques include transformation with T-DNA delivered by *Agrobacterium tumefaciens* or *Agrobacterium rhizogenes* as the transformation agent. Additionally, fusion of plant protoplasts with liposomes containing the DNA to be delivered, direct injection of the DNA, biolistics transformation (microparticle bombardment), or electroporation, as well as other possible methods, may be employed.

In a preferred embodiment of the subject invention, plants will be transformed with genes wherein the codon usage of the protein coding region has been optimized for plants. See, for example, US Patent No. 5380831. Also, advantageously, plants encoding a truncated toxin will be used. The truncated toxin typically will encode about 55% to about 80% of the full length toxin. Methods for creating synthetic *B.t.* genes for use in plants are known in the art (Stewart, 2007).

Regardless of transformation technique, the gene is preferably incorporated into a gene transfer vector adapted to express the *B.t* insecticidal toxin genes and variants in the plant cell by including in the vector a plant promoter. In addition to plant promoters, promoters from a variety of sources can be used efficiently in plant cells to express foreign genes. For example, one may use promoters of bacterial origin, such as the octopine synthase promoter, the nopaline synthase promoter, and the mannopine synthase promoter. Non*-Bacillus-thuringiensis* promoters can be used in some preferred embodiments. Promoters of plant virus origin may be used, for example, the 35S and 19S promoters of Cauliflower Mosaic Virus, a promoter from Cassava Vein Mosaic Virus, and the like. Plant promoters include, but are not limited to, ribulose-1,6-bisphosphate (RUBP) carboxylase small subunit (ssu), beta-conglycinin promoter, phaseolin promoter, ADH (alcohol dehydrogenase) promoter, heat-shock promoters, ADF (actin depolymerization factor) promoter, ubiquitin promoter, actin promoter, and tissue specific promoters. Promoters may also contain certain enhancer sequence elements that may improve the transcription efficiency. Typical enhancers include but are not limited to ADH1-intron 1 and ADH1-intron 6. Constitutive promoters may be used. Constitutive promoters direct continuous gene expression in nearly all cells types and at nearly all times (*e.g*., actin, ubiquitin, CaMV 35S). Tissue specific promoters are responsible for gene expression in specific cell or tissue types, such as the leaves or seeds (*e.g..* zein, oleosin, napin, ACP (Acyl Carrier Protein) promoters), and these promoters may also be used. Promoters may also be used that are active during a certain stage of the plants' development as well as active in specific plant tissues and organs. Examples of such promoters include but are not limited to promoters that are root specific, pollen-specific, embryo specific, corn silk specific, cotton fiber specific, seed endosperm specific, phloem specific, and the like.

Under certain circumstances it may be desirable to use an inducible promoter. An inducible promoter is responsible for expression of genes in response to a specific signal, such as: physical stimulus (*e.g.* heat shock genes); light (*e.g.* RUBP carboxylase); hormone (*e.g.* glucocorticoid); antibiotic (*e.g.* tetracycline); metabolites; and stress (*e.g.* drought). Other desirable transcription and translation elements that function in plants may be used, such as 5' untranslated leader sequences, RNA transcription termination sequences and poly-adenylate addition signal sequences. Numerous plant-specific gene transfer vectors are known to the art.

Transgenic crops containing insect resistance (IR) traits are prevalent in corn and cotton plants throughout North America, and usage of these traits is expanding globally. Commercial transgenic crops combining IR and herbicide tolerance (HT) traits have been developed by multiple seed companies. These include combinations of IR traits conferred by *B.t.* insecticidal proteins and HT traits such as tolerance to Acetolactate Synthase (ALS) inhibitors such as sulfonylureas, imidazolinones, triazolopyrimidine, sulfonanilides, and the like, Glutamine Synthetase (GS) inhibitors such as bialaphos, glufosinate, and the like, 4-HydroxyPhenylPyruvate Dioxygenase (HPPD) inhibitors such as mesotrione, isoxaflutole, and the like, 5-EnolPyruvylShikimate-3-Phosphate Synthase (EPSPS) inhibitors such as glyphosate and the like, and Acetyl-Coenzyme A Carboxylase (ACCase) inhibitors such as haloxyfop, quizalofop, diclofop, and the like. Other examples are known in which transgenically provided proteins provide plant tolerance to herbicide chemical classes such as phenoxy acids herbicides and pyridyloxyacetates auxin herbicides (see WO 2007/053482 A2), or phenoxy acids herbicides and aryloxyphenoxypropionates herbicides (see WO 2005107437 A2, A3). The ability to control multiple pest problems through IR traits is a valuable commercial product concept, and the convenience of this product concept is enhanced if insect control traits and weed control traits are combined in the same plant. Further, improved value may be obtained via single plant combinations of IR traits conferred by a *B.t.* insecticidal protein such as that of the subject invention, with one or more additional HT traits such as those mentioned above, plus one or more additional input traits (*e.g*. other insect resistance conferred by *B.t*.-derived or other insecticidal proteins, insect resistance conferred by mechanisms such as RNAi and the like, nematode resistance, disease resistance, stress tolerance, improved nitrogen utilization, and the like), or output traits (*e.g.* high oils content, healthy oil composition, nutritional improvement, and the like). Such combinations may be obtained either through conventional breeding (breeding stack) or jointly as a novel transformation event involving the simultaneous introduction of multiple genes (molecular stack). Benefits include the ability to manage insect pests and improved weed control in a crop plant that provides secondary benefits to the producer and/or the consumer. Thus, the subject invention can be used in combination with other traits to provide a complete agronomic package of improved crop quality with the ability to flexibly and cost effectively control any number of agronomic issues.

The transformed cells grow inside the plants in the usual manner. They can form germ cells and transmit the transformed trait(s) to progeny plants.

Such plants can be grown in the normal manner and crossed with plants that have the same transformed hereditary factors or other hereditary factors. The resulting hybrid individuals have the corresponding phenotypic properties.

In a preferred embodiment of the subject invention, plants will be transformed with genes wherein the codon usage has been optimized for plants. See, for example, US Patent No. 5,380,831. In addition, methods for creating synthetic *Bt* genes for use in plants are known in the art (Stewart and Burgin, 2007). One non-limiting example of a preferred transformed plant is a fertile maize plant comprising a plant expressible gene encoding a Cry3Aa protein, and further comprising a second plant expressible genes encoding a Cry34Ab/35Ab protein.

Transfer (or introgression) of the Cry3Aa- and Cry34Ab/35Ab-determined trait(s) into inbred maize lines can be achieved by recurrent selection breeding, for example by backcrossing. In this case, a desired recurrent parent is first crossed to a donor inbred (the non-recurrent parent) that carries the appropriate gene(s) for the Cry-determined traits. The progeny of this cross is then mated back to the recurrent parent followed by selection in the resultant progeny for the desired trait(s) to be transferred from the non-recurrent parent. After three, preferably four, more preferably five or more generations of backcrosses with the recurrent parent with selection for the desired trait(s), the progeny will be heterozygous for loci controlling the trait(s) being transferred, but will be like the recurrent parent for most or almost all other genes (see, for example, Poehlman & Sleper (1995) Breeding Field Crops, 4th Ed., 172-175; Fehr (1987) Principles of Cultivar Development, Vol. 1: Theory and Technique, 360-376).

Insect Resistance Management (IRM) Strategies. Roush *et al.,* for example, outlines two-toxin strategies, also called "pyramiding" or "stacking," for management of insecticidal transgenic crops. (The Royal Society. Phil. Trans. R. Soc. Lond. B. (1998) 353, 1777-1786).

On their website, the United States Environmental Protection Agency (epa.gov/oppbppd1/biopesticides/pips/bt_corn_refuge_2006.htm) publishes the following requirements for providing non-transgenic (*i.e., non-B.t.)* refuges (a block of non-Bt crops / corn) for use with transgenic crops producing a single Bt protein active against target pests.
"The specific structured requirements for corn borer-protected Bt (Cry1Ab or Cry1F) corn products are as follows:
Structured refuges:
   20% non-Lepidopteran Bt corn refuge in Corn Belt;
   50% non-Lepidopteran Bt refuge in Cotton Belt
Blocks
   Internal (*i.e.,* within the Bt field)
   External (*i.e.,* separate fields within ½ mile (¼ mile if possible) of the Bt field to maximize random mating)
In-field Strips
   Strips must be at least 4 rows wide (preferably 6 rows) to reduce the effects of larval movement"

In addition, the National Corn Growers Association, on their website:
(ncga.com/insect-resistance-management-fact-sheet-bt-corn)
also provides similar guidance regarding the refuge requirements. For example:
   "Requirements of the Corn Borer IRM:
      - Plant at least 20% of your corn acres to refuge hybrids
      - In cotton producing regions, refuge must be 50%
      - Must be planted within 1/2 mile of the refuge hybrids
      - Refuge can be planted as strips within the Bt field; the refuge strips must be at least 4 rows wide
      - Refuge may be treated with conventional pesticides only if economic thresholds are reached for target insect
      - Bt-based sprayable insecticides cannot be used on the refuge corn
      - Appropriate refuge must be planted on every farm with Bt corn"

As stated by Roush *et al.* (on pages 1780 and 1784 right column, for example), stacking or pyramiding of two different proteins each effective against the target pests and with little or no cross-resistance can allow for use of a smaller refuge. Roush suggests that for a successful stack, a refuge size of less than 10% refuge, can provide comparable resistance management to to about 50% refuge for a single (non-pyramided) trait. For currently available pyramided Bt corn products, the U.S. Environmental Protection Agency requires significantly less (generally 5%) structured refuge of non-Bt corn be planted than for single trait products (generally 20%).

There are various ways of providing the IRM effects of a refuge, including various geometric planting patterns in the fields (as mentioned above) and in-bag seed mixtures, as discussed further by Roush *et al.* (*supra*), and U.S. Patent No. 6,551,962.

The above percentages, or similar refuge ratios, can be used for the subject double or triple stacks or pyramids.

Following are examples that illustrate procedures for practicing the invention. These examples should not be construed as limiting. All percentages are by weight and all solvent mixture proportions are by volume unless otherwise noted. All temperatures are in degrees Celsius.

Unless specifically indicated or implied, the terms "a", "an", and "the" signify "at least one" as used herein.

### EXAMPLES

### Example 1 - Expression and Purification

Construction of expression plasmids encoding Cry34Ab1, Cry35Ab1, and Cry3Aa full-length toxins. Standard cloning methods were used in the construction of *Pseudomonas fluorescens* (*Pf*) expression plasmids engineered to produce a full-length Cry34Ab1, Cry35Ab1, and Cry3Aa1 Cry proteins respectively. Restriction endonucleases from New England BioLabs (NEB; Ipswich, MA) were used for DNA digestion and T4 DNA Ligase from Invitrogen was used for DNA ligation. Plasmid preparations were performed using the Plasmid Midi Kit (Qiagen), following the instructions of the supplier. DNA fragments were purified using the Millipore Ultrafree®-DA cartridge (Billerica, MA) after agarose Tris-acetate gel electrophoresis. The basic cloning strategy entailed subcloning the coding sequences (CDS) of full-length Cry34Ab1, Cry35Ab1, and Cry3Aa1 Cry proteins into pMYC1803 at, for example, *SpeI* and *XhoI* (or *XbaI,* or *HindIII*) restriction sites, respectively, whereby they were placed under the expression control of the *Ptac* promoter and the rrnBT1T2 terminator from plasmid pKK223-3 (PL Pharmacia, Milwaukee, WI), respectively. pMYC1803 is a medium copy plasmid with the RSF1010 origin of replication, a *tetracycline resistance* gene, and a ribosome binding site preceding the restriction enzyme recognition sites into which DNA fragments containing protein coding regions may be introduced (US Patent Application No. 20080193974). The expression plasmid was transformed by electroporation into a *P. fluorescens* strain MB214, recovered in SOC-Soy hydrolysate medium, and plated on Luria broth (LB) medium containing 20 µg/ml tetracycline. Details of the microbiological manipulations are available US Patent Application No. 20060008877, US Patent Application No. 20080193974, and US Patent Application No. 20080058262. Colonies were screened by restriction digestion of miniprep plasmid DNA. Plasmid DNA of selected clones containing inserts was sequenced by contract with a commercial sequencing vendor such as MWG Biotech (Huntsville, AL). Sequence data were assembled and analyzed using the Sequencher™ software (Gene Codes Corp., Ann Arbor, MI).

Growth and expression. Growth and expression analysis in shake flasks production of Cry34Ab1, Cry35Ab1, and Cry3Aa1 toxins for characterization including Bt receptor binding and insect bioassay was accomplished by shake-flask-grown *P. fluorescens* strains harboring expression constructs (*e.g*. clone pMYC2593 for Cry34Ab1, pMYC3122 for Cry35Ab1, and pMYC1334 for Cry3Aa1). Seed cultures grown in LB medium supplemented with 20 µg/ml tetracycline were used to inoculate 200 mL of the same medium with 20 µg/ml tetracycline. Expression of Cry34Ab1, Cry35Ab1, and Cry3Aa1 toxins via the *Ptac* promoter were induced by addition of isopropyl-β-D-1-thiogalactopyranoside (IPTG) after an initial incubation of 24 hours at 30°C with shaking. Cultures were sampled at the time of induction and at various times post-induction. Cell density was measured by optical density at 600 nm (OD₆₀₀).

Cell fractionation and SDS-PAGE analysis of shake flask samples. At each sampling time, the cell density of samples was adjusted to OD₆₀₀ = 20 and 1 mL aliquots are centrifuged at 14,000 x g for five minutes. The cell pellets were frozen at -80°C. Soluble and insoluble fractions from frozen shake flask cell pellet samples were generated using EasyLyse™ Bacterial Protein Extraction Solution (EPICENTRE® Biotechnologies, Madison, WI). Each cell pellet was resuspended in 1 mL EasyLyse™ solution and further diluted 1:4 in lysis buffer and incubated with shaking at room temperature for 30 minutes. The lysate was centrifuged at 14,000 rpm for 20 minutes at 4°C and the supernatant was recovered as the soluble fraction. The pellet (insoluble fraction) was then resuspended in an equal volume of phosphate buffered saline (PBS; 11.9 mM Na₂HPO₄, 137 mM NaCl, 2.7 mM KC1, pH7.4). Samples were mixed 1:1 with 2X Laemmli sample buffer containing β-mercaptoethanol and boiled for 5 minutes prior to loading onto NuPAGE Novex 4-20% Bis-Tris gels (Invitrogen, Carlsbad, CA). Electrophoresis was performed in the recommended XT MOPS buffer. Gels were stained with the SimplyBlue™ Safe Stain according to the manufacturer's (Invitrogen) protocol and imaged using the Typhoon imaging system (GE Healthcare Life Sciences, Pittsburgh, PA).

Inclusion body preparation. Cry protein inclusion body (IB) preparations were performed on cells from *P. fluorescens* fermentations that produced insoluble *B.t.* insecticidal protein, as demonstrated by SDS-PAGE and MALDI-MS (Matrix Assisted Laser Desorption/Ionization Mass Spectrometry). *P. fluorescens* fermentation pellets are thawed in a 37°C water bath. The cells were resuspended to 25% w/v in lysis buffer (50 mM Tris, pH 7.5, 200 mM NaCl, 20 mM EDTA disodium salt (Ethylenediaminetetraacetic acid), 1% Triton X-100, and 5 mM Dithiothreitol (DTT); 5 mL/L of bacterial protease inhibitor cocktail (P8465 Sigma-Aldrich, St. Louis, MO) were added just prior to use. The cells were suspended using a homogenizer at lowest setting (Tissue Tearor, BioSpec Products, Inc., Bartlesville, OK). Lysozyme (25 mg of Sigma L7651, from chicken egg white) was added to the cell suspension by mixing with a metal spatula, and the suspension was incubated at room temperature for one hour. The suspension was cooled on ice for 15 minutes, then sonicated using a Branson Sonifier 250 (two 1- minute sessions, at 50% duty cycle, 30% output). Cell lysis was checked by microscopy. An additional 25 mg of lysozyme was added if necessary, and the incubation and sonication were repeated. When cell lysis was confirmed via microscopy, the lysate was centrifuged at 11,500 x g for 25 minutes (4°C) to form the IB pellet, and the supernatant was discarded. The IB pellet was resuspended with 100 mL lysis buffer, homogenized with the hand-held mixer and centrifuged as above. The IB pellet was repeatedly washed by resuspension (in 50 mL lysis buffer), homogenization, sonication, and centrifugation until the supernatant became colorless and the IB pellet became firm and off-white in color. For the final wash, the IB pellet was resuspended in sterile-filtered (0.22 µm) distilled water containing 2 mM EDTA, and centrifuged. The final pellet was resuspended in sterile-filtered distilled water containing 2 mM EDTA, and stored in 1 mL aliquots at -80°C.

SDS-PAGE analysis and quantification. SDS-PAGE analysis and quantification of protein in IB preparations were done by thawing a 1 mL aliquot of IB pellet and diluting 1:20 with sterile-filtered distilled water. The diluted sample was then boiled with 4X reducing sample buffer [250 mM Tris, pH6.8, 40% glycerol (v/v), 0.4% Bromophenol Blue (w/v), 8% SDS (w/v) and 8% β-Mercapto-ethanol (v/v)] and loaded onto a Novex® 4-20% Tris-Glycine, 12+2 well gel (Invitrogen) run with 1X Tris/Glycine/SDS buffer (Invitrogen). The gel was run for approximately 60 min at 200 volts then stained and distained by following the SimplyBlue™ Safe Stain (Invitrogen) procedures. Quantification of target bands was done by comparing densitometric values for the bands against Bovine Serum Albumin (BSA) samples run on the same gel to generate a standard curve using the Bio-Rad Quantity One software.

Solubilization of inclusion bodies. Ten mL of inclusion body suspensions from P. *fluorescens* clones MR1253, MR1636, and MR832 (containing 50-70 mg/mL of Cry34Ab1, Cry35Ab1, and Cry3Aa1 proteins respectively) were centrifuged at the highest setting of an Eppendorf model 5415C microfuge (approximately 14,000 x g) to pellet the inclusions. The storage buffer supernatant was removed and replaced with 25 mL of 100 mM sodium acetate buffer, pH 3.0, for both Cry34Ab1 and Cry35Ab1, and 100 mM sodium carbonate buffer, pH11, for Cry3Aa1, in a 50 mL conical tube, respectively. Inclusions were resuspended using a pipette and vortexed to mix thoroughly. The tubes were placed on a gently rocking platform at 4°C overnight to extract full-length Cry34Ab1, Cry35Ab1, and Cry3Aa1 proteins. The extracts were centrifuged at 30,000 x g for 30 min at 4°C, and saved the resulting supernatants containing solubilized full-length Cry proteins.

Truncation of full-length protoxins. Full-length Cry35Ab1 and Cry3Aa1 were truncated or digested with chymotrypsin or trypsin to get their chymotrypsin or trypsin core that are an active form the proteins. Specially, the solubilized full-length Cry35Ab1 was incubated with chymotrypsin (bovine pancreas) (Sigma, St. MO) at (50:1 = Cry protein: enzyme, w/w) in the 100 mM sodium acetate buffer, pH 3.0, at 4°C with gentle shaking for 2-3 days, while full-length Cry3Aa1 was incubated with trypsin (bovine pancreas) (Sigma, St. MO) at (20:1 = Cry protein: enzyme, w/w) in the 100 mM sodium carbonate buffer, pH11, at room temperature for 1-3 hours. Complete activation or truncation was confirmed by SDS-PAGE analysis. The molecular mass of full-length Cry35Ab1 and Cry3Aa1 was ≈44 and ≈73 kDa, and their chymotrypsin or trypsin core was ≈40 and ≈55 kDa, respectively. The amino acid sequences of the chymotrypsin Cry35Ab core is provided as SEQ ID NO:5. The amino acid sequences of the trypsin Cry3Aa core is provided as SEQ ID NO:2. Neither chymotrypsin or trypsin core is available for Cry34Ab1; thus the full-length Cry34Ab1 was used for binding assays. The amino acid sequence of the full-length Cry34Ab1 is provided as SEQ ID NO:3.

Purification of truncated toxins. The chymotrypsinized Cry35Ab1 and trypsinized Cry3Aa1 were purified. Specifically, the digestion reactions were centrifuged at 30,000 x g for 30 min at 4°C to remove lipids, and the resulting supernatant were concentrated 5-fold using an Amicon Ultra-15 regenerated cellulose centrifugal filter device (10,000 Molecular Weight Cutoff; Millipore). The sample buffers were then changed to 20 mM sodium acetate buffer, pH 3.5, for both Cry34Ab1 and Cry35Ab1, and to 10 mM CAPS [3-(cyclohexamino)1-propanesulfonic acid], pH 10.5, for Cry3Aa1, using disposable PD-10 columns (GE Healthcare, Piscataway, NJ). The final volumes were adjusted to 15 ml using the corresponding buffer for purification using ATKA Explorer liquid chromatography system (Amersham Biosciences). For Cry35Ab1, buffer A was 20 mM sodium acetate buffer, pH 3.5, and buffer B was buffer A + 1 M NaCl, pH 3.5. A HiTrap SP (5 ml) column (GE) was used. After the column was fully equilibrated using the buffer A, the Cry35Ab1 solution was injected into the column at a flow rate of 5 ml/min. Elution was performed using gradient 0-100% of buffer B at 5 ml/min with 1 ml/fraction. For Cry3Aa1, buffer A was 10 mM CAPS buffer, pH 10.5, and buffer B was 10 mM CAPS buffer, pH 10.5 + 1 M NaCl. A Capto Q, 5 ml (5 ml) column (GE) was used and the all other procedures were similar to that for Cry35Ab1. After SDS-PAGE analysis of the selected fractions to further select fractions containing the best quality target protein, pooled those fractions. The buffer was changed for the purified Cry35Ab1 chymotrypsin core with 20 mM Bist-Tris, pH 6.0, as described above. For the purified Cry3Aa1 trypsin core, the salt was removed using disposable PD-10 columns (GE Healthcare, Piscataway, NJ). The samples were saved at 4°C for later binding assay after being quantified using SDS-PAGE and the Typhoon imaging system (GE) analyses with BSA as a standard.

### Example 2 - Binding Assays

BBMV preparations. Brush border membrane vesicle (BBMV) preparations of insect midguts have been widely used for Cry toxin receptor binding assays. The BBMV preparations used in this invention were prepared from isolated midguts of third instars of the western corn rootworm (*Diabrotica virgifera virgifera* LeConte) using the method described by Wolferberger et al. (1987). Leucine aminopeptidase was used as a marker of membrane proteins in the preparation and Leucine aminopeptidase activities of crude homogenate and BBMV preparation were determined as previously described (Li et al. 2004a). Protein concentration of the BBMV preparation was measured using the Bradford method (1976).

¹²⁵I Labelling. Purified full-length Cry34Ab1, chymotrypsinized Cry35Ab1, and trypsinized Cry3Aa were labeled using ¹²⁵I for homologous and competition binding assays. To ensure the radio-labeling does not abolish the biological activity of the Cry toxins, cold iodination was conducted using NaI followed the instructions of Pierce® Iodination Beads (Pierce Biotechnology, Thermo Scientific, Rockford IL). Bioassay results indicated that iodinated Cry35Ab1 chymotrypsin core remained active against the larvae of the western corn rootworm, but iodination inactivated Cry34Ab1. Radiolabeled ¹²⁵I-Cry34Ab1 did not specifically bind to the insect BBMV, and Cry34Ab1 requires another labeling method to assess membrane receptor binding. Bioassay using cold iodinated Cry3Aa trypsin core is on going and the result will be available in one or two weeks. Radiolabeled ¹²⁵I-Cry35Ab1 and ¹²⁵I-Cry3Aa1 were obtained with Pierce® Iodination Beads (Pierce) and Na¹²⁵I. Zeba™ Desalt Spin Columns (Pierce) were used to remove unincorporated or free Na¹²⁵I from the iodinated protein. The specific radioactivities of the iodinated Cry proteins ranged from 1-5 uCi/ug. Multiple batches of labeling and binding assays were conducted.

Specific binding assays. Specific binding assays were performed using ¹²⁵I-labeled Cry toxins as described previously (Li et al. 2004b). To determine specific binding and estimate the binding affinity (disassociation constant, Kd) and binding site concentration (Bmax) of Cry35Ab1 and Cry3Aa to the insect BBMV, a series of increasing concentrations of either ¹²⁵I-Cry35Ab1 or ¹²⁵I-Cry3Aa were incubated with a given concentration (0.05 mg/ml) of the insect BBMV, respectively, in 150 ul of 20 mM Bis-Tris, pH 6.0, 136.9 mM NaCl, 2.7 mM KC1, supplemented with 0.1 % BSA at room temperature for 60 min with gentle shaking. Toxin bound to BBMV was separated from free toxins in the suspension by centrifugation at 20,000 x g at room temperature for 8 min. The pellet was washed twice with 900 ul of ice-cold the same buffer containing 0.1% BSA. The radioactivity remaining in the pellet was measured with a COBRAII Auto-Gamma counter (Packard, a Canberra company) and considered total binding. Another series of binding reactions were setup at side by side, and a 500-1,000-fold excess of unlabeled corresponding toxin was included in each of the binding reactions to fully occupy all specific binding sites on the BBMV, which was used to determine non-specific binding. Specific binding was estimated by subtracting the non-specific binding from the total binding. The Kd and Bmax values of these toxins were estimated using the specific binding against the concentrations of the labeled toxin used by running GraphPad Prism 5.01 (GraphPad Software, San Diego, CA). The charts were made using either Microsoft Excel or GraphPad Prism programs. The experiments were replicated at least three times. These binding experiments demonstrated that both ¹²⁵I-Cry35Ab1 and ¹²⁵I-Cry3Aa were able to specifically bind to the BBMV (Figure 1A and 1B). ¹²⁵I-Cry35Ab1 and ¹²⁵I-Cry3Aa had a binding affinity Kid=2.31±1.26, 24.0±9.7 (nM), respectively, and a binding site concentration Bmax=31.69±6.38, 146.3±39.9 (pmole/ug BBMV), respectively.

Competition binding assays. Competition binding assays were further conducted to determine if Cry35Ab1 and Cry3Aa share a same set of receptors. For Cry3Aa homologous competition binding assays, increasing amounts (0-5,000 nM) of unlabeled Cry3Aa were first mixed with 5 nM labeled Cry3Aa, and then incubated with a given concentration (0.05 mg/ml) of BBMV at room temperature for 60 min, respectively. The percentages of bound ¹²⁵I-Cry3Aa with BBMV were determined for each of the reactions as compared to the initial specific binding at absence of unlabeled competitor. Heterologous competition binding assays between ¹²⁵I-Cry3Aa and unlabeled Cry35Ab1 alone or unlabeled Cry35Ab1+Cry34Ab1 (1:3 molar ratio) were also performed respectively to identify if they share a same set of receptor(s). This was achieved by increasing the amount of unlabeled Cry35Ab1 alone or the Cry35Ab1+Cry34Ab1 mixture as one or two competitors included in the reactions to compete for the putative receptor(s) on the BBMV with the labeled Cry3Aa. The experiments were replicated at least three times. The experimental results demonstrated that Cry3Aa was able to completely displace itself when the molar concentration increased to 500 nM (100 folds excess ¹²⁵I-Cry3Aa) (Figure 2). However, either Cry35Ab1 alone or the mixture of Cry35Ab1+Cry34Ab1 (1:3) was not able to displace ¹²⁵I-Cry3Aa. These data indicated that Cry35Ab1 alone or Cry35Ab1+Cry34Ab1 (1:3) does not share a receptor with Cry3Aa.

### References:

Bradford, M.M. 1976. A rapid and sensitive method for the quantitation of microgram quantities of protein utilizing the principle of protein-dye binding, Anal. Biochem. 72, 248-254.
Li, H., Oppert, B., Higgins, R.A., Huang, F., Zhu, K.Y., Buschman, L.L., 2004a. Comparative analysis of proteinase activities of Bacillus thuringiensis-resistant and -susceptible Ostrinia nubilalis (Lepidoptera: Crambidae). Insect Biochem. Mol. Biol. 34, 753-762.
Li, H., Oppert, B., Gonzalez-Cabrera, J., Ferré, J., Higgins, R.A., Buschman, L.L. and Zhu, K.Y. and Huang, F. 2004b. Binding analysis of Cry1Ab and Cry1Ac with membrane vesicles from Bacillus thuringiensis-resistant and -susceptible Ostrinia nubilalis (Lepidoptera: Crambidae). Biochem. Biophys. Res. Commun. 323, 52-57.
Wolfersberger, M.G., Luthy, P., Maurer, A., Parenti, P., Sacchi, F., Giordana, B., Hanozet, G.M., 1987. Preparation and partial characterization of amino acid transporting brush border membrane vesicles from the larval midgut of the cabbage butterfly (Pieris brassicae). Comp. Biochem. Physiol. 86A, 301-308.
US Patent Application No. 20080193974. 2008. BACTERIAL LEADER SEQUENCES FOR INCREASED EXPRESSION
US Patent Application No. 20060008877, 2006. Expression systems with sec-system secretion.
US Patent Application No. 20080058262, 2008. rPA optimization.

## Claims

1. A transgenic plant that produces a Cry34Ab1 protein, a Cry35Ab1 protein, and a Cry3Aa insecticidal protein, wherein said Cry35Ab1 protein is at least 95% identical with a sequence selected from the group consisting of SEQ ID NO:4 and SEQ ID NO:5, said Cry3Aa insecticidal protein is at least 95% identical with a sequence consisting of SEQ ID NO:2, and said Cry34Ab1 protein is at least 95% identical with SEQ ID NO:3.

2. The transgenic plant of claim 1, wherein said transgenic plant further produces a fourth insecticidal protein selected from the group consisting of Cry3B and Cry6A.

3. A seed of the transgenic plant according to any of claims 1-2, wherein said seed comprises DNA encoding said Cry34Ab1 protein, said Cry35Ab1 protein and said Cry3Aa protein.

4. A plurality of transgenic plants according to any of claims 1-2.

5. A seed bag or container comprising a plurality of seeds of claim 3.

6. A transgenic plant of any of claims 1-2, wherein said plant is a maize plant.

7. A plant cell of a transgenic plant of any of claims 1-2, wherein said Cry35Ab1 protein is at least 95% identical with a sequence selected from the group consisting of SEQ ID NO:4 and SEQ ID NO:5, said Cry3Aa insecticidal protein is at least 95% identical with a sequence consisting of SEQ ID NO:2, and said Cry34Ab1 protein is at least 95% identical with SEQ ID NO:3.

8. A transgenic plant of any of claims 1-2, wherein said Cry35Ab1 protein comprises a sequence selected from the group consisting of SEQ ID NO:4 and SEQ ID NO:5, said Cry3Aa insecticidal protein comprises a sequence consisting of SEQ ID NO:2, and said Cry34Ab1 protein comprises SEQ ID NO:3.

9. A method of producing the plant cell of claim 7, said method comprising transforming a plant cell with DNA capable of expressing any one of the proteins of claims 1-2.

10. A method of controlling a corn rootworm insect by contacting said insect with a Cry34Ab1 protein, a Cry35Ab1 protein, and a Cry3Aa insecticidal protein.

11. The transgenic plant of claim 2 wherein said Cry3B protein is a Cry3Ba protein and said Cry6A protein is a Cry6Aa protein.

## Patentansprüche

1. Eine transgene Pflanze, die ein Cry34Abl-Protein, ein Cry35Abl-Protein und ein Cry3Aa-insektizides Protein produziert, wobei das Cry35Abl-Protein mindestens 95% identisch mit einer Sequenz ist, ausgewählt aus der Gruppe bestehend aus SEQ ID NO:4 und SEQ ID NO:5, das Cry3Aa-insektizide Protein mindestens 95% identisch ist mit einer Sequenz bestehend aus SEQ ID NO:2, und das Cry34Ab1-Protein mindestens 95% identisch ist mit SEQ ID NO:3.

2. Die transgene Pflanze nach Anspruch 1, wobei die transgene Pflanze zudem ein viertes insektizides Protein produziert, ausgewählt aus der Gruppe bestehend aus Cry3B und Cry6A.

3. Ein Samen der transgenen Pflanze nach irgendeinem der Ansprüche 1-2, wobei der Samen DNS umfasst, die für das Cry34Abl-Protein, das Cry35Abl-Protein und das Cry3Aa-Protein kodiert.

4. Eine Mehrzahl transgener Pflanzen nach irgendeinem der Ansprüche 1-2.

5. Eine Samentasche oder ein Behälter, umfassend eine Mehrzahl an Samen nach Anspruch 3.

6. Eine transgene Pflanze nach irgendeinem der Ansprüche 1-2, wobei die Pflanze eine Maispflanze ist.

7. Eine Pflanzenzelle einer transgenen Pflanze nach irgendeinem der Ansprüche 1-2, wobei das Cry35Abl-Protein mindestens 95% identisch mit einer Sequenz ist, ausgewählt aus der Gruppe bestehend aus SEQ ID NO:4 und SEQ ID NO:5, das Cry3Aa-insektizide Protein mindestens 95% identisch ist mit einer Sequenz bestehend aus SEQ ID NO:2 und das Cry34Ab1-Protein mindestens 95% identisch ist mit SEQ ID NO:3.

8. Eine transgene Pflanze nach irgendeinem der Ansprüche 1-2, wobei das Cry35Abl-Protein eine Sequenz umfasst, ausgewählt aus der Gruppe bestehend aus SEQ ID NO:4 und SEQ ID NO:5, das Cry3Aa-insektizide Protein eine Sequenz umfasst bestehend aus SEQ ID NO:2 und das Cry34Abl-Protein SEQ ID NO:3 umfasst.

9. Ein Verfahren zur Herstellung der Pflanzenzelle nach Anspruch 7, das Verfahren umfassend das Transformieren einer Pflanzenzelle mit DNS, die fähig ist, irgendeines der Proteine nach den Ansprüche 1-2 zu exprimieren.

10. Ein Verfahren zum Überwachen eines Maiswurzelbohrer-Insektes durch in Kontaktbringen des Insektes mit einem Cry34Abl-Protein, einem Cry35Abl-Protein und einem Cry3Aa-insektiziden Protein.

11. Die transgene Pflanze nach Anspruch 2, wobei das Cry3B-Protein ein Cry3Ba-Protein und das Cry6A-Protein ein Cry6Aa-Protein ist.

## Revendications

1. Plant transgénique qui produit une protéine Cry34Ab1, une protéine Cry35Abl et une protéine insecticide Cry3Aa, dans lequel ladite protéine Cry35Ab1 est au moins à 95 % identique à une séquence choisie dans le groupe consistant en la SEQ ID N°4 et la SEQ ID N°5, ladite protéine insecticide Cry3Aa est au moins à 95 % identique à une séquence consistant en la SEQ ID N°2, et ladite protéine Cry34Abl est au moins à 95 % identique à la SEQ ID N°3.

2. Plant transgénique selon la revendication 1, dans lequel ledit plant transgénique produit en outre une quatrième protéine insecticide choisie dans le groupe consistant en Cry3B et Cry6A.

3. Semence du plant transgénique selon l'une quelconque des revendications 1 à 2, dans lequel ladite semence comprend de l'ADN codant ladite protéine Cry34Ab1, ladite protéine Cry35Abl et ladite protéine Cry3Aa.

4. Pluralité de plants transgéniques selon l'une quelconque des revendications 1 à 2.

5. Sachet ou récipient de semence comprenant une pluralité de semences selon la revendication 3.

6. Plant transgénique selon l'une quelconque des revendications 1 à 2, dans lequel ledit plant est un plant de maïs.

7. Cellule végétale d'un plant transgénique selon l'une quelconque des revendications 1 à 2, dans laquelle ladite protéine Cry35Abl est au moins à 95 % identique à une séquence choisie dans le groupe consistant en la SEQ ID N°4 et la SEQ ID N°5, ladite protéine insecticide Cry3Aa est au moins identique à 95 % à une séquence consistant en la SEQ ID N°2, et ladite protéine Cry34Abl est au moins identique à 95 % à la SEQ ID N°3.

8. Plant transgénique selon l'une quelconque des revendications 1 à 2, dans lequel ladite protéine Cry35Abl comprend une séquence choisie dans le groupe consistant en la SEQ ID N°4 et la SEQ ID N°5, ladite protéine insecticide Cry3Aa comprend une séquence consistant en la SEQ ID N°2, et ladite protéine Cry34Abl comprend la SEQ ID N°3.

9. Procédé de production de la cellule végétale selon la revendication 7, ledit procédé comprenant la transformation d'une cellule végétale avec un ADN capable d'exprimer l'une quelconque des protéines selon les revendications 1 à 2.

10. Procédé de contrôle d'une chrysomèle des racines de maïs par la mise en contact dudit insecte avec une protéine Cry34Ab1, une protéine Cry35Abl et une protéine insecticide Cry3Aa.

11. Plant transgénique selon la revendication 2, dans lequel ladite protéine Cry3B est une protéine Cry3Ba et ladite protéine Cry6A est une protéine Cry6Aa.
